# EUROPEAN PATENT APPLICATION

(11) **EP 4 512 393 A1**
(43) Date of publication of application: **26.02.2025**
(21) Application number: 24172667.8
(22) Date of filing: 26.04.2024
(51) Int. Cl.: A61K 31/19, A61P 29/00, A61P 31/04, A61P 31/12

(54) **COMBINATION OF SODIUM DICHLOROACETATE AND VALPROIC ACID SALTS FOR THE TREATMENT OF VIRAL AND BACTERIAL INFECTIONS AND INFLAMMATORY DISEASES**

(30) Priority: 22.08.2023 LT 2023532
(71) Applicant: Lietuvos sveikatos mokslu universitetas, 44307 Kaunas (LT)
(72) Inventor: Lesauskaite, Vaiva, 44307 A. Mickeviciaus str. 9 (LT); Stakisaitis, Donatas, 44307 A. Mickeviciaus str. 9 (LT); Kapocius, Linas, 48283 Zemales str. 3 (LT); Gecys, Dovydas, 44307 A. Mickeviciaus str. 9 (LT); Valanciute, Angelija, 44307 A. Mickeviciaus str. 9 (LT); Balnyte, Ingrida, 44307 A. Mickeviciaus str. 9 (LT); Ugenskiene, Ruta, 44307 A. Mickeviciaus str. 9 (LT); Vaitkevicius, Arunas, 01118 J. Basanaviciaus 9b-16 (LT); Tatarunas, Vacis, 44307 A. Mickeviciaus str. 9 (LT); Tamosuitis, Tomas, 44307 A. Mickeviciaus str. 9 (LT); Urboniene, Daiva, 44307 A. Mickeviciaus str. 9 (LT); Mickiene, Aukse, 44307 A. Mickeviciaus str. 9 (LT); Damanskiene, Eligija, 44307 A. Mickeviciaus str. 9 (LT)
(74) Representative: Zaboliene, Reda

(57) **Abstract**

The invention provides preclinical *in vivo* and *in vitro* and clinical trials *in vitro* data as well as the expression of genes related to the inflammation and immune response to the medicinal product under development, using differential gene expression analysis of sequencing data. The evaluation of the effect of the new DCA-VPA combination based on research data leads to the conclusion that treatment with the DCA-VPA combination under development influences gene regulation disorders caused by viral and bacterial infection in the body and is suitable for correcting or inhibiting pathological processes at the molecular level. Based on the results obtained from this advanced technology, a new medicinal product consisting of a combination of dichloroacetate and valproic acid medicinal products is being developed for the treatment of viral and bacterial infections and inflammatory diseases, which can improve the course of the treated disease, accelerate patient recovery, survival, quality of life and reduce healthcare costs.

## Description

### FIELD OF THE INVENTION

The invention relates to the use of a combination of sodium dichloroacetate (DCA) and valproic acid (VPA) salts (DCA-VPA) for the adjunctive therapeutic treatment of viral and bacterial infections, as well as for inflammatory diseases.

### STATE OF THE ART

The combination of preparations is used for the treatment of infectious diseases as additional therapy to obtain a therapeutic effect. Preparations that make up rational combinations have different mechanisms of action that suppress infection by restoring the impaired metabolism of the body and immune cells. This can lead to a faster recovery, prevent immunity impairment and organ damage, i.e. reduce the risk of unfavourable disease course and disease progression. The combination uses well-known drugs of different classes with a well-known safety profile. The component of the combination sodium dichloroacetate (DCA) suppresses glycolysis and the development of metabolic acidosis. By suppressing the production of lactic acid, DCA also inhibits the release of pro-inflammatory cytokines. Valproic acid (VPA) has an immunomodulating effect that suppresses inflammatory reactions caused by viruses or bacteria. VPA activates DCA transport into cells via the SLC5A8 transporter. Such synergism of the compounds of combination is based on the mechanism of pharmacological action i. e. VPA activates intracellular expression of the SLC5A8 gene by demethylation mechanisms, which enhances the transport of DCA into the mitochondria of the cell. The search for new drugs in the treatment of severe infections and suppressing the development of metabolic disorders is an important area of infectious disease research The development of combinations of medicinal products as new medicines for the treatment of various diseases is supported by inventions.

US9556113 is for the use of a combination of dichloroacetate and oxamate and combinations of dichloroacetate and synthesised oxamate prodrugs in the treatment of cancers of various localisations. The patent describes the anti-cancer efficacy and the mechanisms of pharmacological action of the combination of two drugs that complement each other, i.e. act synergistically. These preparations can be used as primary or adjunctive therapy (separately or as a combination in one preparation) and in parallel with systemic chemotherapy.

US8609724 describes the use of DCA and chemical equivalents thereof in the treatment of cancer for the purpose of inducing apoptosis or reducing resistance to apoptosis. It is authorized for 10 to 100 mg/kg sodium DCA in combination with other pro- apoptotic agents or chemotherapy in the treatment of non-small cell lung cancer, breast cancer and glioblastoma.

EP1708692 B1 describes the use of DCA to improve cardiac contractile function and coronary blood flow after ischemia-reperfusion. Combination with DCA allows a reduction in the dose of inotropic drugs (dopamine, epinephrine, ephedrine, phenylephrine, dobutamine) after improvement of cardiac index indicators. DCA amount of 50 mg/kg is administered by intravenous infusion to newborns, children, or adults; when used in combination with inotropic drugs, it protects the myocardium from damage. DCA improves recovery of myocardial function and metabolism after episodes of ischemia (cardiac surgery, hemorrhagic shock, hypoxia).

US20140066482 and EP2026787 disclose the treatment of insulin-independent diabetes by combining the registered agents repaglinide and metformin. Repaglinide promotes the release of insulin from pancreatic β-cells, while metformin reduces insulin resistance, suppresses glucose production in the liver, and increases the sensitivity of muscle and fat tissue to insulin. The compounds in the combination differ in chemical formula, pharmacokinetics and mechanisms of pharmacological action. The patented combination formulation produces a synergistic effect of the two components in the treatment of insulin-independent diabetes.

### BRIEF DESCRIPTION OF THE INVENTION

The aim of the invention is to provide a novel use of a pharmaceutical combination which comprises an effective amount of (a) dichloroacetate (DCA) and (b) valproic acid (VPA) or a pharmaceutically acceptable salt thereof for the treatment of viral and bacterial infections and inflammatory diseases. A pharmaceutically acceptable salt of dichloroacetate is sodium dichloroacetate. A pharmaceutically acceptable salt of valproic acid is sodium valproate or magnesium valproate. The above combination additionally contains pharmaceutically acceptable excipients. The final formulation is available in any pharmaceutical form, such as a capsule, tablet or solution.

The invention describes a combination of DCA-VPA for use in the treatment of viral and bacterial infections and inflammatory diseases. This combination is characterized by a new pharmacological mechanism of action of DCA and VPA: both drugs inhibit inflammatory reactions, have an immuno-modulating effect, promote the development of anti-inflammatory biological pathways during infectious inflammation, inhibit biological pathways associated with pro-inflammatory reactions, inhibit aerobic glycolysis.

Application of the DCA-VPA combination results in a synergistic anti-inflammatory effect of the constituents of the test preparation, which is manifested by VPA activation of DCA transport into cells via the SLC5A8 carrier.

The combination of active substances is intended for use in the treatment of viral or bacterial infections and inflammatory diseases such as typhoid fever and paratyphoid fever, other infections caused by salmonella, shigellosis, other intestinal infections caused by bacteria, infections caused by pathogenic Escherichia coli, enterocolitis caused by Clostridium difficile, amebiasis, tuberculosis, non-intestinal yersiniasis, pertussis, meningococcal infection, streptococcal sepsis, other sepsis, legionellosis, staphylococcal infection, Lyme disease, atypical viral infections of the central nervous system, mosquito-borne viral encephalitis, tick-borne viral encephalitis, herpes simplex virus infections, shingles, measles, acute hepatitis, acute hepatitis C, chronic viral hepatitis, human immunodeficiency virus (HIV) disease, cytomegalovirus disease, infectious mononucleosis, malaria, leishmaniasis, african trypanosomiasis, Chagas disease, toxoplasmosis, flu and pneumonia, COVID disease, *Helicobacter pylori* infection, human papillomavirus infection, SARS, MERS infections, infections caused by Gram-positive bacteria, infections caused by Gram-negative bacteria, inflammatory bowel diseases.

A combination of active ingredients comprising (a) sodium dichloroacetate (DCA) and (b) valproic acid (VPA) or a pharmaceutically acceptable salt thereof is administered either as a combined preparation or the individual active ingredients are administered in combination as separate medicinal products. The combination is administered as an adjunctive therapy together with the known specific therapy of the infection or the inflammation caused by infection. The treatment of infectious diseases with the above combination of active substances is applied in preclinical and clinical trials.

### BRIEF DESCRIPTION OF THE DRAWINGS

Figure 1. Effect of DCA-VPA on Slc5a8 gene expression in mouse thymocytes.
Figure 2. Effects of DCA-VPA on rat thymocytes CD⁴⁺_IL17⁺ for the expression of thymocyte population.
Figure 3. Effect of DCA-VPA on rat thymocytes CD⁴_CD27⁻ for the expression of thymocyte population.
Figure 4. Effect of DCA-VPA on the expression of genes related to inflammation or immune response in murine thymocytes
Figure 5. Effects of DCA-VPA on murine thymocyte genes by inhibiting the IL-17 signaling pathway.
Figure 6. Effect of DCA-VPA on the expressions of cytokine genes involved in the pathogenesis of Covid-19 in murine thymocytes .
Figure 7. Effects of DCA-VPA treatment on the pathophysiological mechanisms of inflammation and immune response in T-lymphocytes from healthy patients related to inflammatory and immune response genes.
Figure 8. Effect of DCA-VPA treatment on the pathophysiological mechanisms of inflammation and immune response of stimulated T-lymphocytes in healthy patients associated with changes in gene expression.
Figure 9. Effect of DCA-VPA on biological pathways in T-lymphocytes from adult patients with severe form of COVID-19.

### DETAILED DESCRIPTION OF THE INVENTION

Severe infections disrupt the systemic metabolism, which can be the main reason for the death of the organism. DCA and VPA are investigational medicinal products with anti-inflammatory effects in viral and bacterial infections, which are characterized by the restoration of inflammation-induced metabolic disturbances and pharmacological effects that correct the immune response. Preclinical and clinical studies have shown that the combination of DCA and VPA has an anti-inflammatory effect by inhibiting the release of pro-inflammatory mediators, suppressing the expression of genes that promote inflammation and immune reactions in T-lymphocytes, and increasing the expression of genes that suppress inflammatory reactions caused by infection.

### Anti-inflammatory and immune-regulating effects of dichloroacetate

Sodium dichloroacetate (DCA) is inhibitor of pyruvate dehydrogenases kinase (PDK). DCA increases the activity of pyruvate dehydrogenase (PDH) and its complex (PDHC), promotes glucose oxidation, and inhibits glycolysis by inhibiting PDK activity. Various acquired metabolic and immune system dysfunction diseases are characterized by an increased pathological expression of PDK, which leads to the suppression of PDHC, which regulates the most important metabolic processes, including lactic acid production, the tricarboxylic acid cycle (TCA), lipid metabolism, and the release of inflammatory mediators. DCA is a small molecule drug commonly used in the treatment of diseases associated with mitochondrial defects and increased congenital or acquired lactic acid production. DCA inhibits the accumulation of lactic acid in tissues, blood and inflammatory cells. DCA significantly restored TCA metabolite levels to control levels and improved liver function in sepsis by activating PDHC. DCA is an investigational drug in clinical trials that normalises oxidative stress and increases resistance to infection. Decreased intracellular PDHC activity and increased blood lactic acid concentrations are biomarkers of disease severity and important targets for therapeutic approaches to the pathogenesis of inflammation or the metabolic processes of sepsis.

DCA activates the alpha subunit of PDH (PDHA1), which catalyses the oxidative dehydrogenation of pyruvate to acetyl-CoA, and inhibits lactic acid production. Decreased PDH activity is characteristic of peripheral blood mononuclear cells in severe infectious inflammation and sepsis. Inactivation of PDHA1 is associated with metabolic reprogramming and lactic acid overproduction. Inactivation of PDH during infection is detected in peripheral blood mononuclear cells, skeletal muscle cells, vascular endothelial cells, and macrophages. Inflammation induced by lipopolysaccharide (LPS) stimulation increased PDK mRNA expression 24-fold in mouse digital extensor muscle cells and reduced PDHC activity by 65%. PDHC activity in skeletal muscle cells of septic rats was reduced by -70%. PDHC activity in peripheral blood mononuclear cells from sepsis patients was significantly lower than in healthy cells. The final product of glycolysis is lactic acid, its increased concentration in the blood is an independent indicator of poor prognosis for sepsis, COVID-19, malaria, and other infections. The increase in the concentration of endogenous lactic acid partially explains the clinical association of its blood level with the risk of acute renal failure and the incidence of liver damage in sepsis.

DCA is metabolised to glycine in rodents and humans. Glycine deficiency is a disorder that occurs in severe infections and sepsis, when the expression of the gene responsible for glycine synthesis is reduced in liver cells. The administration of glycine and cysteine restores glutathione synthesis and mitochondrial oxidative mechanisms in HIV-infected patients. Glycine administration reduces liver damage and systemic inflammatory reactions in a rat model of sepsis. The potential effect of DCA on glycine levels may indicate that glycine, a metabolite of DCA, may have therapeutic effects.

In a murine model of sepsis, DCA treatment was found to abolish cardiovascular shock, reduce hepatocellular injury, hyperlactatemia, hyperglycaemia, reverse immunosuppression and metabolic paralysis, improve bacterial clearance, and significantly increases animal survival during sepsis.

DCA regulates the anabolic and catabolic energy supply for the immune cells. In the acute phase of hyperinflammation, the lack of acetyl-CoA leads to anaerobic glycolysis, reorganisation of TCA and lipid metabolism, and the accumulation of succinate and citrate, as well as lactic acid, which are inflammatory mediators. Lactic acid can activate the Toll-like receptor 4 (TLR4) and stimulate the activation of nuclear factor-kappa B (NF-κB) and the subsequent release of inflammatory mediators.

Studies have shown the effect of DCA, i.e. reducing lactic acid levels early in sepsis reduces the extent of organ dysfunction, the progression of fibrosis and the development of chronic kidney disease. Clinical studies have shown that reducing lactic acid in early sepsis improves the prognosis of the disease. Treatment of sepsis with DCA can control inflammation. Lactic acid activates the innate immune response through TLR-mediated enhancement of NF-κB transcriptional activity and pro-inflammatory gene expression in macrophages. DCA inhibits the activation of monocytes, dendritic cells, and macrophages. Lactic acid, as a proinflammatory metabolite, affects macrophage polarisation, enhances pro-inflammatory factor production, and stimulates vascular endothelial growth factor expression, whereas inhibition of lactic acid production blocks TLR4 signalling and attenuates pro-inflammatory factor production. Knockdown of PDK1 inhibited LPS-induced polarization of bone marrow macrophages from M2 to M1 phenotype in septic mice, resulting in decreased levels of the inflammatory mediators such as interleukin IL-6, IL-12, and IL-1β. Treatment with DCA may result in a polarisation of M1 macrophages to an M2 phenotype, thereby reducing the secretion of inflammatory mediators. PDK1 is required for M1 macrophage polarisation and the production of TNFα, IL-1β and IL-6. Inhibition of PDK activated AMPKα1 in macrophages. Inhibition of PDK activated AMPKα1 in macrophages. AMPK is a metabolic signaling molecule involved in the formation of the M2 macrophage phenotype, it also increases the activity of Akt and CREB, and inhibits the activity of NF-κB. One of the mechanisms underlying the increase in inflammation is pro-inflammatory mediators such as interferon-gamma (IFN-γ).

When compared to control septic animals, DCA infusion decreases plasma lactic acid concentration and glycolytic activity. Intravenous DCA has been safely used for patients in critical conditions.

The expression and activity of pyruvate dehydrogenase kinase 1 (PDK1) is continuously increased during the inflammatory anabolic phase of sepsis. This leads to the dysfunction of mitochondrial respiration and cellular bioenergetics observed during sepsis. Therefore, PDK is a potential therapeutic target.

The peroxisome proliferator-activated receptor alpha (PPARα), a transcription factor, is considered to be one of the key regulators of fatty acid oxidation. The expression of PPARα is altered during sepsis. The biological activity of PPARα in cells is significantly reduced in a polymicrobial sepsis model. PPARα activation improved hepatic lipid metabolism and survival during sepsis, indicating the protective nature of this pathway during sepsis.

PDK creates a metabolic profile that activates Th17 cells. DCA reduces the production of pro-inflammatory cytokines in rat models of arthritis, colitis and encephalomyelitis mainly by inhibiting Th17 cell proliferation and promoting regulatory T cell differentiation.

DCA is a promising drug for the treatment of various infections. The replication of *Mycobacteroides massiliense* in macrophages is dependent on host PDK activity. *M*. *massiliense* infection induced a metabolic change in macrophages, with increased glycolysis and decreased oxidative phosphorylation, and DCA treatment reversed this metabolic reprogramming of *M*. *massiliense-*infected macrophages and limited intracellular bacterial replication, promoted autophagy, and inhibited the bacterial proliferation in the phagolysosomes. The pharmacological effects of DCA may represent a new therapeutic strategy for controlling virulent *M*. *massiliense* infections. DCA inhibits the progression of enterovirus A71 infection. Molecular docking results indicated that antiseptic-antibacterial solutions containing dichloroacetate anion showed the most significant inhibition of *Escherichia colli; Klebsiella aerogene*/*Enterobacter aerogenes; Klebsiella pneumoniae; Proteus vulgaris; Pseudomonas aeruginosa, Streptococcus pyogenes,* and *Streptococcus pneumoniae* bacteria that cause skin and soft tissue infections. Cells isolated from kidney biopsies from diabetic patients were more frequently infected with SARS-CoV-2 compared to cells from non-diabetic patients, while DCA reduced SARS-CoV-2 infection in diabetic kidney cells. DCA may be a promising new compound for the treatment of *Salmonella enteric* serovar *Typhimurium* infection. Seasonal influenza A virus A/WSN/33 (H1N1) induced severe respiratory distress syndrome in mice after 6 days, and DCA treatment inhibited the development of this pathology. DCA regulates the activity of pyruvate metabolism in *T. gondii,* suggesting that DCA could be used to treat toxoplasmosis. In malaria, lactic acidosis can be safely treated with DCA. This adjunctive therapy of hyperlactatemia in severe malaria is considered to improve survival by "buying time" until antimalarial drugs start to take effect. Lactic acidosis, a hallmark of severe malaria, is an independent predictor of death. Prolonged hyperlactatemia leads to arrhythmias, kidney damage, respiratory failure, central nervous system dysfunction and other organ damage.

### Anti-inflammatory and immune-regulating effects of valproic acid

Recent bioinformatic techniques enable adaptation of known drugs to new therapeutic indications, such as for COVID-19. One such drug is valproic acid (2-n-propyl-pentanoic acid; VPA). VPA is a histones deacetylase (HDAC) inhibitor. VPA is an antiepileptic drug also being studied as an immunity modulator. VPA has been used in clinical practice for several decades; its therapeutic concentration in blood serum and safety profile is well known, making it an attractive drug to investigate for non-registered therapeutic indications. VPA is being studied in the treatment of various viral infections.

VPA metabolite 4-en-VPA-CoA forms a stable interaction with SARS-CoV2 RNA polymerase nsP12 and can specifically inhibit the target. SARS-CoV-2 virus protein central protease Mpro (3CLpro) is a target for antiviral drugs designed as inhibitors of viral 3CLpro. HDAC inhibitors bind tightly to the active part of the crystallographic structure of viral Mpro. HDAC2 activity can be modulated by selective binding of VPA to the catalytic site. VPA decreased HDAC2 levels in prefrontal cortex tissue of male rats. SARS-CoV-2 protease NSP5 can affect mitochondria by interacting with HDAC2. inhibitors of HDAC can be anti-inflammatory drugs.

SARS-CoV-2 entry into the cell requires ACE2 and expression of transmembrane serine protease 2 (TMPRSS2) receptor. Human lung tissue, alveolar epithelial cells type I and II, arterial and venous endothelial cells, cardiomyocytes, arterial smooth muscle cells, expressing ACE2 are the targets of the SARS-CoV-2 virus. VPA decreases ACE2 levels in endothelial cells. TMPRSS2 is highly expressed in lung tissue cells. TMPRSS2 is a promising therapeutic target for COVID-19. VPA decreases TMPRSS2 expression in cells.

VPA as a multifunctional cellular regulator of the innate and adaptive immune system reduces macrophage infiltration in various models of inflammation. VPA significantly attenuated the regulation of profibrotic and proinflammatory genes, macrophage infiltration in the kidney as well as significantly reduced cigarette smoke-induced neutrophil influx in the Balb/c female mouse lung inflammation model by acting as an endopeptidase inhibitor and has the potential to act as a medicine in inflammatory lung diseases by inducing a reduction in neutrophil infiltration in bronchoalveolar lavage fluid.

VPA inhibited M1 macrophages proliferation, stimulated M2 macrophage accumulation in the Wistar male rat lung injury model, reduced inflammation and improved tissue repair. VPA was able to impair acute lung MAP kinase activation in a sublethal model of hemorrhagic shock and reduced pulmonary neutrophil infiltration. The production of TNF-α, IL-1α, IL-1β, IL-1RA, IL-6, IL-7, IL-10, IL-12p40, IL-12p70, IFN-γ, and TGF-α were decreased after exposure to VPA in LPS-activated dendritic cells of female BALB/c mice. A reduced ability of immune cells to induce a proinflammatory response was found after VPA treatment, suggesting new therapeutic options for the management of septic shock. VPA reduced the clinical progression of Coxsackie virus B3 (CVB3)-induced myocarditis and the mortality of CVB3-induced myocarditis in male BALB/c mice, diminished the percentage of Th17 cells in the spleen, increased the amount of Treg cells, mitigated the expression of IL-17A, and increased the concentration of IL-10 in the blood serum and heart tissue of CVB3-infected mice also suppressed the differentiation of Th17 cells, and promoted the differentiation of Treg cells.

In the NIH3T3/BL6 murine model of postoperative conjunctival scar inflammation, VPA suppresses the synthesis of CXCL1, IL-5, IL-6, and tissue NF-κB2 p100 proteins. VPA reduced macrophage infiltration, apoptotic cell death and caspase 3 activation, a volume of tissue damage, improved active recovery after spinal cord injury in male rats by inhibiting MMP-9 activity. The improvement in disease was associated with VPA-induced mitigation of histological signs of inflammation, and suppression of pro-inflammatory cytokines IFN-γ and IL-6 in an acute colitis model. In a female C57BL/6 mouse model, VPA lessened CD4+ T-lymphocyte infiltrates and it was associated with caspase 3-mediated apoptosis.

VPA reduced the levels of inflammatory cytokines and reactive oxygen species and mitigated organ damage in rats elicited by LPS-induced septic shock by decreasing TNF-α and myeloperoxidase levels in the lung tissue of male rats. The VPA treatment improved early survival, lung, liver and brain function in polytrauma also hemorrhagic shock in male rat models. VPA inhibited leukocyte migration into the peritoneal cavity by 92% in a peritonitis-induced male rat model, reducing TNF-α, IL-1β, IL-6, and reactive oxygen species (ROS) generation with an effect similar to that of indomethacin. A significant increase in blood levels of IL-10 and TGF-β mRNA was found in VPA-treated male rats in the rat renal ischemia/ reperfusion injury model also a direct correlation between IL-1β and TNF-α mRNA expression and IL-10 with TGF-β levels, indicating anti-inflammatory VPA effect. Histopathological studies demonstrated reduced renal ischemic changes and reduced serum creatinine in VPA-treated animals.

VPA significantly inhibited the replication of enveloped viruses and the synthesis of RNA and viral protein, indicating that VPA can interfere with the viral cycle at various stages of viral infection. VPA suppressed vesicular stomatitis virus infection, decreased TNF-α, and IL-6 production in bone marrow macrophages of female BALB/c mice. VPA polarised murine macrophage cell line RAW264.7 and primary female bone marrow macrophages (BMM) in C57BL/6 and BALB/c mice from a pro-inflammatory M1 toward an anti-inflammatory M2 phenotype.

When studied in a model of LPS-induced macrophage activation, VPA inhibited macrophage T helper 1 (Th1) effector, enhanced the activation of Th2 effector cells, influenced macrophage function by inhibiting the production of IL-12 and TNF-α, stimulated the expression of IL-10. Effect of VPA on LPS-induced inflammatory response in murine RAW 264.7 macrophage-like cells suggests that VPA reduces LPS-induced NF-κB-dependent transcriptional activity through attenuated PI3K/Akt/MDM2 activation and suppressed p53 expression.

The levels of CCL2, VEGF-A, IL-15 decreased in VPA-treated fibroblasts and VPA inhibited the induction of CCL5, and VEGF-A and the level of NF-κB2 p100 in the presence of TNF-α. VPA in dengue virus (DENV) infected human monocytes decreased secretion of IL-8, IL-1b, IL-6, TNF-α and IL-10 in macrophages. Studies showed that VPA modulates the cytokine storm associated with DENV disease and prevents severe disease progression. Cell proliferation of T CD8+ lymphocytes from healthy blood donors treated with VPA was reduced.

Virus replication and survival depend on energy produced by the cell's mitochondria, so antiviral therapy may include medicines that alter the mitochondrial energy metabolism. Mitochondria have their own functions in activating antiviral and anti-inflammatory processes. VPA reduces oxidative stress by enhancing the enzymatic antioxidant system. VPA can activate the SLC5A8 gene through the regulation of DNA methylation, and increase its expression in cells. SLC5A8 has a physiological function in the transport of short-chain fatty acids into the cell and regulates mitochondrial metabolism; SLC5A8 is a transporter of DCA into the cell. SLC5A8 activity is characterized by immunomodulatory effects by blocking the development of dendritic cells, thereby making SLC5A8 essential for immune homeostasis by releasing cytokines. SLC5A8 induces cell apoptosis by inhibiting pyruvate-dependent HDACs. Inhibition of the SLC5A8 gene is associated with methylation of DNA and treatment with DNA demethylating agents such as VPA increases SLC5A8 gene expression. VPA via mitochondria can polarise the pro-inflammatory M1 phenotype of macrophages to the anti-inflammatory M2, which cannot induce the differentiation of naïve T CD4+ into a Th1 profile, favoring a Th2 phenotype. SLC5A8 regulates mitochondrial metabolism by participating in the mitochondrial β-oxidation pathway.

Most of the energy for pro-inflammatory-immune cells comes from aerobic glycolysis. Glucose uptake and glycolysis are essential for the rapid growth and proliferation of virus-activated T cells. Increased glucose levels create favorable conditions for the progression of SARS-CoV-2 infection. VPA lowers blood glucose levels in animals and humans.

VPA has antiplatelet and antithrombotic effects. Clinical use of VPA in the treatment of epilepsy is associated with a lower risk of thrombosis, myocardial infarction, and stroke. Thus, VPA could be a potential alternative for the prevention of thrombotic events during severe infections based on improved endogenous fibrinolysis. The antithrombotic effect of VPA may significantly correlate with its effect on suppression of the immune response.

### Characterization of the interaction of DCA and VPA salt combinations in mixtures or solutions

Sodium dichloroacetate (NaDCA ) is an electrolyte and is completely ionised in an aqueous solution. It is a salt of a strong acid (Cl₂HCOOH) and a strong base (NaOH), so ion hydrolysis does not occur in an aqueous solution, the medium remains neutral, and new ionic bonds are not formed. Theoretical calculations related to the activity of ions in solution show that there is no interaction between salt combinations, and this does not affect the biological efficacy of anions. The latter depends only on the concentration of anions in the medium.

The above-mentioned characteristic is also applicable to sodium valproate solution. This solution consists of a weak salt of valproic acid (C₃H₇)₂CHOOH and a strong base (NaOH). The systematic name of VPA is 2-propylpentanoic acid and hydrolysis of the anion occurs as follows:

(C₃H₇)₂CHCOO-(aq) + H₂O(s) ↔ (C₃H₇)₂CHCOOH(aq) + OH(aq) Formula I

The decrease in anion concentration caused by hydrolysis is insignificant and does not affect the biological activity at all. The aqueous solution obtained from the combination of sodium dichloroacetate and sodium valproate is a homogeneous mixture of ions. There is a weak chemical interaction between the ions in this mixture, similar to what happens when salts are in separate solutions. The interaction of the anions in the mixture has no effect on the biological activity, that is, the biological activity is determined only by their concentration. It was observed that the above-mentioned combinations of solid ground salts do not exhibit solid phase interionic transitions at room temperature. These mixtures consisting of cations and anions have a biological activity that is influenced only by the ratio of mixed salts. This suggests that a pharmaceutical formulation can be developed using these salt combinations. Both in solid mixtures and in aqueous solutions, the solubility of the magnesium salts of valproic acid is lower compared to the corresponding sodium salts of valproic acid. However, salt ion hydrolysis processes are slightly more noticeable only in magnesium salt solutions, which are obtained from weak bases and weak acids. Calculations show that this hydrolysis has a minimal effect on the initial ion concentration and does not significantly change the concentrations or biological activity of the existing anion. Currently, there are already several registered pharmaceutical formulations of sodium and magnesium valproate solutions and salts.

### Sequencing study of genes related to inflammation and immune response in murine thymocytes and adult male patients T-lymphocytes

Sequencing gene data quality was assessed using MultiQC v1.13. The 3' nucleotide sequences of the adapters and sequences with a length of less than 15 nucleotides and a quality score of less than 25 were removed using Cutadapt v1.9.1. The human genome (GRCh38.p13) was downloaded from the Ensembl database. The remaining sequences were aligned to the human genome with the STAR 2.1.3 tool. The gene expression matrix was obtained using FeatureCounts v3.15. Sequence expression was normalized using the upper quartile method, and genes with a cumulative expression between samples less than 50 were removed. Differential gene expression analysis was performed with DESeq2 v3.15, p-values were adjusted using the Benjamin-Hochberg method. Expression analysis of a biological pathway enrichment was performed using the DAVID server.

### Enrichment analysis of genes related to inflammation and immune response in DCA-VPA-treated T-lymphocytes from healthy adult patients

T-lymphocytes from eight healthy men were exposed *in vitro* to a combination of 2 mM VPA and 5 mM DCA for 24 hours. The data were compared to control (untreated T-lymphocytes). Gene expression data from treated and control conditions were normalized and log-transformed.

The Enrichplot v1.2 and ClusterProfiler v4.8.2 R packages were used to analyze the Gene Set Enrichment Analysis (GSEA) results. GSEA was performed using established algorithms to calculate enrichment scores and *p*-values for each pathway or gene set of interest. Gene set annotations were obtained from Gene Ontology (GO), KEGG and Reactome databases. Examples of the effects of DCA-VPA on the expression of genes related to inflammation and immune response in T-lymphocytes in biological KEGG pathways of viral and bacterial infections are shown in Table 1.

**Table 1. Enrichment analysis data from a sequence-reading study on the effects of DCA-VPA on the expression of genes related to inflammation and immune response in male T-lymphocytes**

| **Pathway name: Code** | **Amount of affected genes (*n*)** | ***p* meaning** | **Effect of DCA-VPA combination on gene expression** | **Pathway enrichme nt (times)** |
|---|---|---|---|---|
| **Defense response to bacterium:** GO:0042742 | 21 | 4.62E-10 | **Upregulates:** *CCL20, CEBPB, IRF8, IRGM, MICA, PPBP* | 5.93 |
| | | | **Downregulates:** *CLEC4E, CXCL13, FCGR1A, IL10, ISG15, LYZ, MYD88, NOD1, NOD2, OAS1, OAS2, LC11A1, SYK, TLR4, TNFRSF1A* | |
| **Defense response to Gram-positive bacterium:** GO:0050830 | 16 | 1.00E-09 | **Upregulates:** *TNF* | 8.23 |
| | | | **Downregulates:** *C5AR1, HAVCR2, IL12A, IL17A, IL17F, IL7R, IL18, IL27RA, IL6R, LYZ, MYD88, NOD1, NOD2, TLR2, TNFRSF14* | |
| **Defense response to Gram-negative bacterium:** GO:0050829 | 12 | 1.66E-07 | **Upregulates** *IL23A, IRGM* | 8.47 |
| | | | **Downregulates:** *CASP1, CD4, IL17A, IL17F, IL23R, IL6R, LYZ, SLC11A1, TLR4, TNFRSF14* | |
| **Virus receptor activity:** GG:0001618 | 9 | 2.37E-05 | **Upregulates** *CD81* | 7.58 |
| | | | **Downregulates:** *CCR5, CD209, CD4, CD80, CD86, ICAM1, TNFRSF4, TNFRSF14* | |
| **Viral protein interaction with cytokine and cytokine receptor:** hsa04061 | 49 | 1.13E-47 | **Upregulates** *CCL20, CCR10, IL18R1, IL20, IL20RB, PPBP, TNF, TNFRSF10A* | 16.39 |
| | | | **Downregulates:** *ACKR3, CCL2, CCL5, CCL7, CCL13, CCL18, CCL19, CCL22, CCL23, CCL24, CCL28, CCR1, CCR2, CCR3, CCR5, CCR6, CCR8, CSF1, CSF1R, CX3CR1, CXCL1, CXCL2, CXCL3, CXCL5, CXCL9, CXCL10, CXCL11, CXCL12, CXCL13, CXCR2, CXCR3, IL2, IL2RA, IL2RB, IL6R, IL10, IL18, IL18RAP, TNFRSF1A, TNFSF10, TNFRSF14* | |
| **Inflammatory bowel disease:** hsa05321 | 30 | 1.0E-27 | **Upregulates** *IFNG, IL18R1, IL23A, JUN, NFATC1, RELA, RORA, RORC, STAT3, STAT4, TGFB1, TNF* | 15.44 |
| | | | **Downregulates:** *HLA-DMA, HLA-DPA1, IFNGR2, IL10, IL12A, IL12RB1, IL13, IL17A, IL17F, IL18, IL18RAP, IL2, IL22, IL23R, IL4, NOD2, TLR2, TLR4* | |
| **Measles:** hsa05162 | 36 | 1.87E-23 | **Upregulates** *CBLB, CD28, CDK2, CHUK, FADD, IFNB1, IKBKB, IRF3, JAK1, JUN, MAP3K7, MAPK8, RELA, STAT3, TRAF6, TYK2* | 8.67 |
| | | | **Downregulates:** *CD209, EIF2AK2, FAS, IFIH1, IL12A, IL2, IL2RA, IL2RB, IRF7, IRF9, JAK3, MX1, MX2, MYD88, OAS1, OAS2, TLR2, TLR4, TLR7, TP53* | |
| **Influenza A:** hsa05164 | 38 | 2.86E-22 | **Upregulates** *CHUK, FADD, IFNB1, IFNG, IKBKB, IRF3, JAK1, MAPK1, RELA, TNF, TNFRSF10A, TYK2* | 7.44 |
| | | | **Downregulates:** *CASP1, CCL2, CCL5, CIITA, CXCL10, EIF2AK2, FAS, HLA-DMA, HLA-DPA1, ICAM1, IFIH1, IFNGR2, IL12A, IL18, IRF7, IRF9, MX1, MX2, MYD88, NLRP3, OAS1, OAS2, TLR4, TLR7, TNFRSF1A, TNFSF10* | |
| **Epstein-Barr virus infection:** hsa05169 | 40 | 1.55E-21 | **Upregulates** *CDK2, CHUK, FADD, HLA-G, IFNB1, IKBKB, IRF3, JAK1, JUN, MAP2K3, MAP3K7, MAPK8, NFKB2, RELA, RUNX3, STAT3, TNF, TRAF6, TYK2* | 6.63_ |
| | | | **Downregulates:** *BLNK, BTK, CXCL10, EIF2AK2, FAS, FCER2, HLA-DMA, HLA-DPA1, ICAM1, IRF7, IRF9, ISG15, JAK3, LYN, MYC, MYD88, OAS1, OAS2, SYK, TLR2, TP53* | |
| **Coronavirus disease** - **COVID-19:** hsa05171 | 42 | 4.21E-21 | **Upregulates** *C3, CHUK, CSF3, IFNB1, IKBKB, IRF3, JAK1, JUN, MAP3K7, MAPK1, MAPK8, PRKCG, RELA, STAT3, TNF, TYK2* | 6.06 |
| | | | **Downregulates:** *C3AR1, C5AR1, CASP1, CCL2, CXCL10, EIF2AK2, IFIH1, IL12A, IL2, IL6R, IRF9, ISG15, MX1, MX2, MYD88, NLRP3, OAS1, OAS2, STING1, SYK, TLR2, TLR4, TLR7, TLR8, TNFRSF1A, TRAF6* | |
| **Tuberculosis:** hsa05152 | 37 | 1.83E-20 | **Upregulates** *C3, CEBPB, FADD, HSPD1, IFNB1, IFNG, IL23A, IRAK2, JAK1, MAPK1, MAPK8, RELA, TGFB1, TNF, TRAF6* | 6.88 |
| | | | **Downregulates:** *CD14, CD209, CD74, CIITA, CLEC4E, CLEC7A, FCGR1A, HLA-DMA, HLA-DPA1, IFNGR2, IL10, IL12A, IL18, ITGAM, ITGB2, MYD88, NOD2, SYK, TLR1, TLR2, TLR4, TNFRSF1A* | |
| **Hepatitis B:** hsa05161 | 34 | 4.45E-19 | **Upregulates** *CDK2, CHUK, EGR3, ELK1, FADD, IFNB1, IKBKB, IRF3, JAK1, JUN, MAP2K3, MAP3K7, MAPK1, MAPK8, NFATC1, NFATC3, PRKCG, RELA, STAT3, STAT4, TGFB1, TNF, TRAF6, TYK2* | 7.02 |
| | | | **Downregulates:** *FAS, IFIH1, IRF7, JAK3, MMP9, MYC, MYD88, TLR2, TLR4, TP53* | |
| **Toxoplasmosis** : hsa05145 | 28 | 9.28E-18 | **Upregulates** *CHUK, IFNG, IKBKB, IRGM, JAK1, MAP2K3, MAP3K7, MAPK1, MAPK8, RELA, STAT3, TGFB1, TNF, TYK2* | 8.36_ |
| | | | **Downregulates:** *CCR5, CD40LG, CIITA, HLA-DMA, HLA-DPA1, IFNGR2, IL10, IL12A, LY96, MYD88, TLR2, TLR4, TNFRSF1A, TRAF6* | |
| **Leishmaniasis:** hsa05140 | 24 | 1.53E-17 | **Upregulates** *C3, ELK1, IFNG, JAK1, JUN, MAP3K7, MAPK1, PTGS2, RELA, TGFB1, TNF* | 10.43 |
| | | | **Downregulates:** *FCGR1A, HLA-DMA, HLA-DPA1, IFNGR2, IL10, IL12A, IL4, ITGAM, ITGB2, MYD88, TLR2, TLR4, TRAF6* | |
| **Pertussis:** hsa05133 | 22 | 2.59E-15 | **Upregulates** *C3, IL23A, IRF3, IRF8, JUN, MAPK1, MAPK8, RELA, TNF* | 9.69 |
| | | | **Downregulates:** *CASP1, CD14, CXCL5, IL10, IL12A, ITGAM, ITGB2, LY96, MYD88, NLRP3, NOD1, TLR4, TRAF6* | |
| **Hepatitis C:** hsa05160 | 29 | 1.03E-14 | **Upregulates** *TNF, RELA, MAPK1, JAK1, IKBKB, CHUK, IRF3, CD81, FADD, IFNB1, STAT3, TYK2, IFNG, CDK2, PPARA* | 6.18 |
| | | | **Downregulates:** *MX1, EIF2AK2, MX2, TNFRSF1A, OAS2, TP53, OAS1, TRAF6, IFIT1, MYC, CXCL10, IRF7, FAS, IRF9* | |
| **Chagas disease:** hsa05142 | 24 | 1.44E-14 | **Upregulates** *C3, CHUK, FADD, IFNB1, IFNG, IKBKB, JUN, MAPK1, MAPK8, RELA, TGFB1, TNF* | 7.87 |
| | | | **Downregulates:** *CCL2, CCL5, FAS, IFNGR2, IL10, IL12A, IL2, MYD88, TLR2, TLR4, TNFRSF1A, TRAF6* | |
| **Malaria:** hsa05144 | 18 | 2.66E-14 | **Upregulates** *CD81, CSF3, IFNG, MET, TGFB1, TNF* | 12.05_ |
| | | | **Downregulates:** *CCL2, CD40LG, ICAM1, IL10, IL12A, IL18, ITGB2, MYD88, THBS1, TLR2, TLR4, VCAM1* | |
| **Human cytomegalovir us infection:** hsa05163 | 31 | 3.13E-12 | **Upregulates** *CHUK, ELK1, FADD, HLA-G, IFNB1, IKBKB, IRF3, JAK1, MAPK1, NFATC1, NFATC3, PRKCG, PTGS2, RELA, STAT3, TNF, VEGFA* | 4.61 |
| | | | **Downregulates:** *CCL2, CCL5, CCR1, CCR3, CCR5, CXCL12, CXCR2, FAS, IL1R1, IL6R, MYC, STING1, TNFRSF1A, TP53* | |
| **Legionellosis:** hsa05134 | 17 | 4.53E-12 | **Upregulates** *C3, HSPD1, NFKB2, RELA, TNF* | 9.98 |
| | | | **Downregulates:** *CASP1, CD14, CXCL1, CXCL2, CXCL3, IL12A, IL18, ITGAM, ITGB2, MYD88, TLR2, TLR4* | |
| **Yersinia infection:** hsa05135 | 24 | 1.05E-11 | **Upregulates** *CDC42, CHUK, IFNB1, IKBKB, IRF3, JUN, MAP2K3, MAP3K7, MAPK1, MAPK8, NFATC1, NFATC3, RELA, TNF, TRAF6* | 5.86 |
| | | | **Downregulates:** *CASP1, CCL2, CD4, IL10, IL18, IL2, MYD88, NLRP3, TLR4* | |
| **Amoebiasis:** hsa05146 | 20 | 1.10E-10 | **Upregulates** *IFNG, PRKCG, RELA, TGFB1, TNF* | 6.56 |
| | | | **Downregulates:** *CD14, CD1B, CD1C, CD1D, CXCL1, CXCL2, CXCL3, IL10, IL12A, IL1R1, IL1R2, ITGAM, ITGB2, TLR2, TLR4* | |
| **Human immunodeficie ncy virus 1 infection:** hsa05170 | 25 | 1.50E-08 | **Upregulates** *CHUK, FADD, HLA-G, IFNB1, IKBKB, IRF3, JUN, MAP2K3, MAP3K7, MAPK1, MAPK8, NFATC1, NFATC3, PRKCG, RELA, TNF, TRAF6* **Downregulates:** *CCR5, CD4, FAS, MYD88, STING1, TLR2, TLR4, TNFRSF1A* | 3.95 |
| **Shigellosis:** hsa05131 | 26 | 7.03E-08 | **Upregulates** *C3, CDC42, CHUK, IFNB1, IKBKB, IRF3, JUN, MAP3K7, MAPK1, MAPK8, RELA, TNF, TRAF6, UBE2N* | 3.52 |
| | | | **Downregulates:** *CASP1, CCL5, CD14, IL18, IL1R1, MYD88, NLRP3, NOD1, STING1, TLR4, TNFRSF1A, TP53* | |
| **African trypanosomiasis:** hsa05143 | 11 | 7.79E-08 | **Upregulates** *IFNG, PRKCG, TNF* | 9.95 |
| | | | **Downregulates:** *FAS, ICAM1, IDO1, IL10, IL12A, IL18, MYD88, VCAM1* | |
| ***Salmonella* infection:** hsa05132 | 26 | 8.24E-08 | **Upregulates** *CDC42, CHUK, FADD, IKBKB, JUN, MAP2K3, MAP3K7, MAPK1, MAPK8, RELA, TNF, TNFRSF10A, TRAF6* | 3.49 |
| | | | **Downregulates:** *CASP1, CD14, IL18, LY96, MYC, MYD88, NLRP3, NOD1, PTPRC, TLR2, TLR4, TNFRSF1A, TNFSF10* | |
| ***Epithelial cell signaling in Helicobacter pyloriinfection:*** hsa05120 | 14 | 1.10E-07 | **Upregulates** *CDC42, CHUK, IKBKB, JUN, MAPK8, MET, RELA* | 6.69 |
| | | | **Downregulates:** *CCL5, CXCL1, CXCL2, CXCL3, CXCR2, LYN, NOD1* | |
| **Pathogenic *Escherichia coli* infection:** hsa05130 | 21 | 1.46E-06 | **Upregulates** *CDC42, CHUK, FADD, IKBKB, JUN, MAP3K7, MAPK1, MAPK8, RELA, TNF, TNFRSF10A, TRAF6* | 3.57 |
| | | | **Downregulates:** *CASP1, FAS, IL18, IL1R1, MYD88, NLRP3, TLR4, TNFRSF1A, TNFSF10* | |
| ***Herpessimplex* virus 1infection:** hsa05168 | 35 | 3.55E-06 | **Upregulates** *C3, CHUK, FADD, HLA-G, IFNB1, IFNG, IKBKB, IRF3, JAK1, MAP3K7, RELA, TNF, TYK2* | 2.37 |
| | | | **Downregulates:** *CCL2, CCL5, CD74, EIF2AK2, FAS, HLA-DMA, HLA-DPA1, IFIH1, IFNGR2, IL12A, IRF7, IRF9, MYD88, OAS1, OAS2, STING1, SYK, TLR2, TNFRSF14, TNFRSF1A, TP53, TRAF6* | |
| **Viralmyocarditis:** hsa05416 | 11 | 9.24E-06 | **Upregulates** *CAV1, CD28, HLA-G* | 6.13 |
| | | | **Downregulates:** *CD40LG, CD80, CD86, HLA-DMA, HLA-DPA1, ICAM1, ITGB2, PRF1* | |
| **Human papillomavirus infection:** hsa05165 | 26 | 1.58E-05 | **Upregulates** *CDC42, CDK2, CHUK, FADD, HLA-G, IFNB1, IKBKB, IRF3, JAK1, MAPK1, PRKCZ, PTGS2, RELA, TNF, TYK2, VEGFA* | 2.63 |
| | | | **Downregulates:** *EIF2AK2, FAS, IRF9, ISG15, MX1, MX2, SPP1, THBS1, TNFRSF1A, TP53* | |
| ***Staphylococcus aureus*infection:** hsa05150 | 11 | 0.00058 | **Upregulates** *C3* | 3.83 |
| | | | **Downregulates:** *IL10, HLA-DMA, HLA-DPA1, ICAM1, ITGAM, C5AR1, ITGB2, FPR1, C3AR1, FCGR1A* | |

Enrichment analysis of the effects of DCA-VPA showed that DCA-VPA treatment upregulated or downregulated the expression of specific genes involved in inflammation or immune response in the biological pathways of bacterial or viral infections (Table 1).

DCA-VPA treatment upregulated gene expression of *C3, CAV1, CBLB, CCL20, CCR10, CD28, CD81, CDC42, CDK2, CEBPB, CHUK, CSF3, EGR3, ELK1, FADD, HLA-G, HSPD1, IFNB1, IFNG, IKBKB, IL18R1, IL20, IL20RB, IL23A, IRAK2, IRF3, IRF8, IRGM, JAK1, JUN, MAP2K3, MAP3K7, MAPK1, MAPK8, MET, MICA, NFATC1, NFATC3, NFKB2, PPARA, PPBP, PRKCG, PRKCZ, PTGS2, RELA, RORA, RORC, RUNX3, STAT3, STAT4, TGFB1, TNF, TNFRSF10A, TRAF6, TYK2, UBE2N, VEGFA.*
The genes whose expression is downregulated by DCA-VPA include *ACKR3, BLNK, BTK, C3AR1, C5AR1, CASP1, CCL13, CCL18, CCL19, CCL2, CCL22, CCL23, CCL24, CCL28, CCL5, CCL7, CCR1, CCR2, CCR3, CCR5, CCR6, CCR8, CD14, CD1B, CD1C, CD1D, CD209, CD4, CD40LG, CD74, CD80, CD86, CIITA, CLEC4E, CLEC7A, CSF1, CSF1R, CX3CR1, CXCL1, CXCL10, CXCL11, CXCL12, CXCL13, CXCL2, CXCL3, CXCL5, CXCL9, CXCR2, CXCR3, EIF2AK2, FAS, FCER2, FCGR1A, FPR1, HAVCR2, HLA-DMA, HLA-DPA1, ICAM1, IDO1, IFIH1, IFNGR2, IFNGR2, IL10, IL12A, IL 12RB1, IL13, IL 17A, IL 17F, IL18, IL18RAP, IL1R1, IL1R2, IL2, IL22, IL23R, IL27RA, IL2RA, IL2RB, IL4, IL6R, IL6R, IL7R, IRF7, IRF9, ISG15, ISG15, ITGAM, ITGB2, JAK3, LC11A1, LY96, LYN, LYZ, MYC, MYD88, MMP9, MX1, MX2, NLRP3, NOD1, NOD2, OAS1, OAS2, PRF1, PTPRC, SYK, SLC11A1, SPP1, STING1, THBS1, TLR1, TLR2, TLR4, TLR7, TLR8, TNFRSF14, TNFRSF1A, TNFRSF4, TNFSF10, TP53, TRAF6, VCAM1.*

The results of the GSEA analysis show a significant effect of DCA-VPA treatment on the expression and regulation of genes related to inflammation and immunity, a therapeutic effect of the preparation on important cellular processes related to inflammation caused by viral or bacterial infection. The detected changes in T-lymphocyte gene expression suggest that DCA-VPA treatment can modulate inflammation and immune response, and the therapeutic effect can improve the course of infection.

The DCA-VPA combination, according to the invention, is intended for use in treatment of viral or bacterial infections or inflammatory diseases consisting of the group including typhoid and paratyphoid (International Classification of Diseases code - A01), other salmonella infections (A02), shigellosis (A03), other intestinal infections caused by bacteria (A04), infections caused by pathogenic *Escherichia coli* (A04.0 - A04.4), *Clostridium difficile* enterocolitis (A04.7), amoebiasis (A06), tuberculosis (A15 - A19), extraintestinal yersiniosis (A28.2), pertussis (A37), meningococcal infection (A39), streptococcal sepsis (A40), other sepsis (A41), legionellosis (A48.1), staphylococcal infection (A49.0), Lyme disease (A69.2), atypical viral infections of the central nervous system (A81), mosquito-borne viral encephalitis (A83), tick-borne viral encephalitis (A84), herpes viruses (*herpes simplex*) infections (B00), herpes zoster (B02), measles (B05), acute hepatitis (B16), acute hepatitis C (B17.1), chronic viral hepatitis (B18), human immunodeficiency virus (HIV) disease (B20 - B24), cytomegalovirus disease (B25), infectious mononucleosis (B27), malaria (B50 - B54), leishmaniasis (B55), African trypanosomiasis (B56), Chagas disease (B57), toxoplasmosis (B58), influenza and pneumonia (J09 - J18), COVID disease (U07.1), *Helicobacter pylori* infection (B96.81), human papillomavirus infection, SARS, MERS infections, infections caused by Gram-positive bacteria, infections caused by Gram-negative bacteria, inflammatory bowel diseases.

### Synergistic effect of DCA and VPA on intracellular transport of dichloroacetate anion

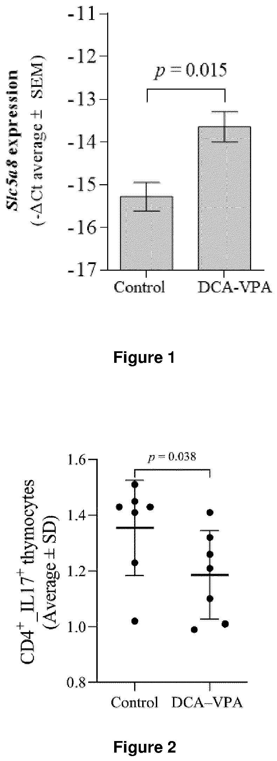

Figure 1 shows the effect of DCA-VPA treatment on *Slc5a8* gene expression *in vivo* in thymocytes of male Mus Musculus mice. Eleven mice were studied (6-7 weeks old, 6 - control and 5 - treated with the combination). Mice were given an aqueous solution of DCA-VPA combination (DCA 100 mg/kg and VPA 150 mg/kg/day) for two weeks. The treatment with the combination statistically significantly increased the expression of *Slc5a8* in mouse thymocytes compared to control. Statistical significance was calculated according to the Mann-Whitney *U* test. SLC5A8 is a sodium- and chloride-dependent monocarboxylate carrier that transports the dichloroacetate anion into cells. Inhibition of the SLC5A8 gene is associated with DNA methylation and VPA inhibits HDAC activity by inducing DNA demethylation, and increases *Slc5a8* expression in cells. This effect is synergistic, with VPA enhancing the transport of DCA into cells and thereby potentiating the pharmacological effects of DCA.

### DCA-VPA effect on CD4⁺_IL17 and CD4⁺_CD27⁻_IFNγ⁺ rat thymocytes population size

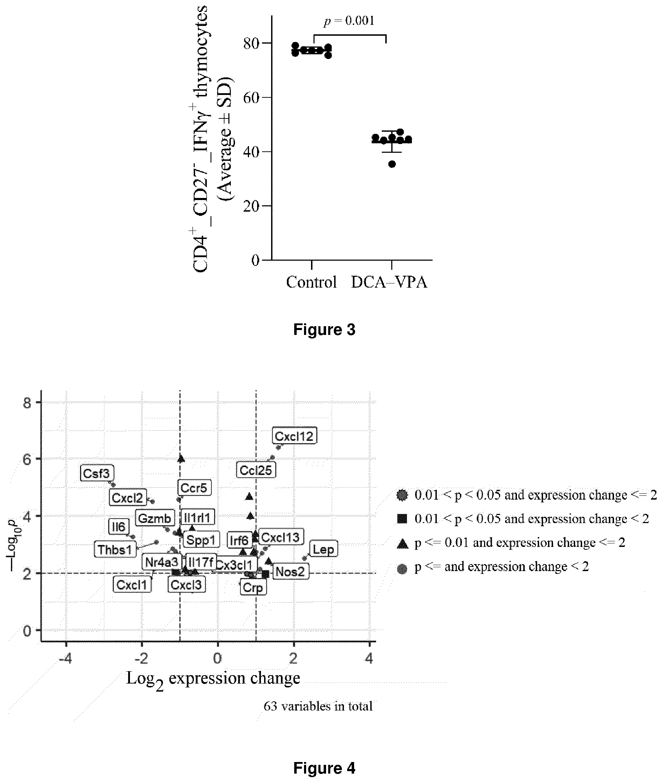

Figures 2 and 3 present data from flow cytometry of male rat thymocytes, it is the effect of DCA-VPA on populations of male rat thymocytes, CD4⁺_IL17 and CD4⁺_CD27⁻_IFNγ⁺. Thymocytes from the following groups of rats were analysed: control group (n = 7), DCA-VPA-treated group (n = 7). Rats were given an aqueous solution of DCA-VPA combination (DCA 100 mg/kg and VPA 150 mg/kg/day) for two weeks.

The count of CD4+_IL17 thymocytes was statistically significantly reduced in male rats treated with DCA-VPA for two weeks compared to matched controls (Figure 2). Th17 lymphocytes, NK, CD⁸⁺ and γδT cells can secrete IL-17. IL-17 stimulates neutrophil pro-inflammatory responses during inflammatory diseases, causes several pro-inflammatory diseases due to dysregulation of IL-17 and IL-22. Therefore, Th17-mediated inhibiting the proinflammatory response may be an effective strategy for treating cytokine storm. Fedratinib is useful for inhibiting Th17 cells and IL-17 expression in rat models. The levels of Th17-related pro-inflammatory cytokines IL-17, IL-15, IL-6, TNF, and IFN are significantly elevated during SARS-CoV-2 infection and correlate with the severity of COVID-19. The amount of IL-17 in patients with SARS-CoV-2 or Middle East respiratory syndrome (MERS) infection with acute respiratory distress syndrome (ARDS) is higher than in normally infected patients.

The population of CD4⁺_CD27⁻_IFN-γ⁺ thymocytes secreting IFN-y was statistically significantly lower in DCA-VPA-treated rats for two weeks compared with the corresponding control (Figure 3). IFN-y is the only type II IFN produced by a variety of lymphocyte cells, including CD4⁺ and CD8⁺ T cells, B cells, and NK cells. IFN-y can also be synthesised by other immune cells, such as monocytes, macrophages, DCs and neutrophil granulocytes. However, T cells and NK cells are the main sources of this cytokine. Because of its diverse activities, IFN-y is difficult to classify as a pro- or anti-inflammatory cytokine. COVID-19 patients have elevated serum level of IFN-γ that is like level in SARS-CoV and MERS infections, but no significant differences in IFN-y levels were found between severe and mild COVID-19 patients. CD4⁺ T cells from individuals with severe symptoms were found to secrete lower amounts of IFN-y compared to individuals with mild symptoms, suggesting that SARS-CoV-2 infection may play an important role in IFN-γ secretion and T cell function.

### DCA-VPA effect on the expression of genes related to inflammation and immune response in murine thymocytes

Figure 4 shows the effect of DCA-VPA treatment on the expression of genes related to inflammation and immune response *in vivo* in thymocytes of male *Mus Musculus* mice. Eleven mice were studied (6-7 weeks old, 6 - control and 5 - treated with the combination). Mice were given an aqueous solution of DCA-VPA combination (DCA 100 mg/kg and VPA 150 mg/kg/day) for two weeks. The effect of DCA-VPA treatment was calculated by comparing gene expression between treated and control mice. Significant changes in the expression of identified genes at p < 0.05 are shown below.

DCA-VPA treatment significantly increased the expression of genes related to inflammation and immune response: *Acad11, Ackr4, Adad1, Art3, Bmp4, Bmp7, C3, Ccl19, Ccl25, Cebpa, Ciita, Cmklr1, Crp, Cx3cl1, Cxcl11, Cxcl12, Cxcl13, Dexi, Egfr, Egr3, Glce, Ifitm3, Il2, Il21, Il23a, Il27, Il33, Il7, Irf6. Lep, Lyz1, Lrp1, Nos2, Pparg, Rnf128,* and *Socs1.*

DCA-VPA treatment significantly reduced the expression of genes related to inflammation and immune response: *Ccl2, Ccl3, Ccr5, Cdkn1a, Cebpb, Clec4e, Csf3, Ctla4, Cxcl1, Cxcl2, Cxcl3, Gzmb, H2-Q7, Icos, Il17f, Il18r1, Il1rl1, Il6, Nr4a3, Ptgs2, Rel, Rn18s, Rora, Spp1, Thbs1, Tnfsf11,* and *Vegfa.* Data from mouse gene sequencing demonstrated that DCA-VPA treatment has an anti-inflammatory effect.

### DCA-VPA combination inhibitory effect on the IL-17 signaling pathway in mouse thymocytes

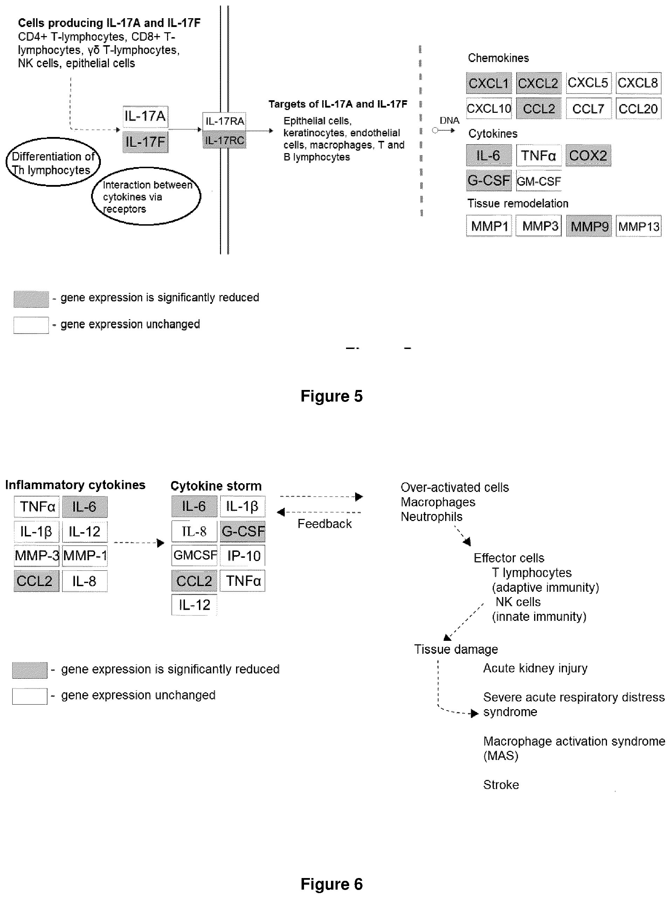

Sequencing of genes related to inflammation and immune response in mice showed that DCA-VPA treatment inhibits the biological pathway of IL-17 signaling by inhibiting IL-17F and IL-17RC gene, chemokine CXCL1, CXCL2, CCL2 gene and cytokine IL-6, COX2, and G-CSF gene expression (Figure 5).

DCA-VPA inhibits the IL-17RC and IL-RC circuits in the IL-17 signaling pathway (Figure 5). The IL-17 receptor (IL-17R) consists of two chains: specific IL-17RC and common IL-17RA as a homodimer or IL-17A/F as a heterodimer. Homodimers of IL-17F bind to the same receptor. Human cells have IL-17RA/C and respond to IL-17A or IL-F with similar induction of inflammatory genes.

Proinflammatory cytokines or chemokines are produced during infection, such as TNF, IL-6, type 1 IFN, chemokine ligand CCL2, which triggers the migration of T-lymphocytes, monocytes and macrophages to the site of infection. The CCL2 is also released during viral infections when IL-1 and TNF-α are produced. This exacerbates inflammation and attracts NK and T cells that secrete IFN-y to the site of inflammation. The ACE2-Ang II axis, which leads to macrophage infiltration and the release of cytokines IL-6 and CCL2, is associated with endothelial dysfunction, thrombin generation and poor fibrinolysis. SARS-CoV-2 significantly increases cellular expression of CXCL1, CXCL2, CCL2, IL-1, TNF-α, and IL-2.

DCA-VPA significantly inhibits the cytokine granulocyte colony-stimulating factor (G-CSF) gene expression. G-CSF and chemokine CCL2 levels were significantly higher in intensive care unit-treated COVID-19 patients than in non-hospitalized patients.

DCA-VPA inhibits cyclooxygenase-2 (COX-2) gene expression. The inducible COX-2 enzyme regulates the expression levels of many serum proteins, including proinflammatory cytokines, during viral infections. Hyperexpression of COX-2 has been described in patients who died from H5N1 infection and is associated with poor clinical outcomes in viral infections. Production of COX-2 metabolites can cause coagulopathy and hyperinflammation in patients with severe COVID-19. SARS-CoV-2 induced changes in COX-2 in various human and murine cell culture systems that could be associated with lung inflammation and disease severity. COX-2 is associated with increased mortality in patients with COVID-19. High levels of COX-2 reduce the production of the endogenous antiviral compound arachidonic acid, making patients more susceptible to COVID-19.

### DCA-VPA effect on the biological pathway of COVID-19 by altering the expression of genes related to inflammation and immune response in murine thymocytes

DCA-VPA inhibited gene expression of the inflammatory cytokines IL-6 and CCL2 (MCP-1) in T-lymphocytes, as well as the cytokines IL-6, G-CSF, MCP-1, which are important in the pathogenesis of cytokine storm (Figure 6). Interleukin IL-6 is important in controlling the inflammatory acute-phase protein response; it induces IL-1 and TNF-α, and IL-6 secretion, regulates STAT3 activation, promotes T cell growth and supports T cell function, together with TGF-β and IL-21 promote Th17 formation in cells. Elevated level of IL-6 increases blood vessel permeability, allowing inflammation to spread rapidly. Dysregulation of IL-6 is associated with tissue cell apoptosis and necrosis. IL-6 regulates lymphocyte depletion, necrosis and chemotaxis of neutrophils, what is essential for the pathophysiology of COVID-19. SARS-CoV-2 activates macrophages, which induce necrosis of lymphocytes producing IL-6 rather than TNF-α or IL-1. According to systematic reviews and meta-analyses, more than 50% of patients with COVID-19 had elevated IL-6 level. Interleikine IL-6 levels were higher in patients with cytokine storm or acute respiratory distress syndrome compared with healthy controls; IL-6 levels were increased fourfold in COVID-19 and multiple systemic and extrapulmonary diseases.

DCA-VPA significantly inhibits the expression of chemokine ligand 2 (CCL2), also known as MCP-1. The CCL2 controls the chemotaxis of monocytes, their migration to vascular endothelium, infiltration, and differentiation into macrophages. It is a factor in many inflammatory disorders, such as COVID-19, ischemic heart disease and stroke. The levels are elevated during SARS-CoV-2 infection and are associated with respiratory failure, stroke, and are associated with increased levels of IL-1, IL-6, TNF-α, and intercellular adhesion molecule-1. Among severe COVID-19 patients, those with increased CCL2 expression were more likely to develop kidney failure and had a worse prognosis than those with lower CCL2 levels.

DCA-VPA inhibits matrix metalloproteinase-9 (MMP-9) gene expression. Gene expression of MMP-9 was increased in the lung tissue of COVID-19 patients. MMP-9 released from neutrophils promotes inflammation and the breakdown of the alveolar-capillary barrier, promotes the migration of inflammatory cells to the lung tissue and its destruction. The concentration of MMP-9 in plasma is directly proportional to the risk of respiratory failure and is associated with mortality in COVID-19 patients. Severe COVID-19 has many features similar to sepsis, and MMP-9 is considered a biomarker in sepsis patients.

### Effects of DCA-VPA treatment on the pathophysiological mechanisms of T-lymphocyte-related inflammation and immune response in healthy patients

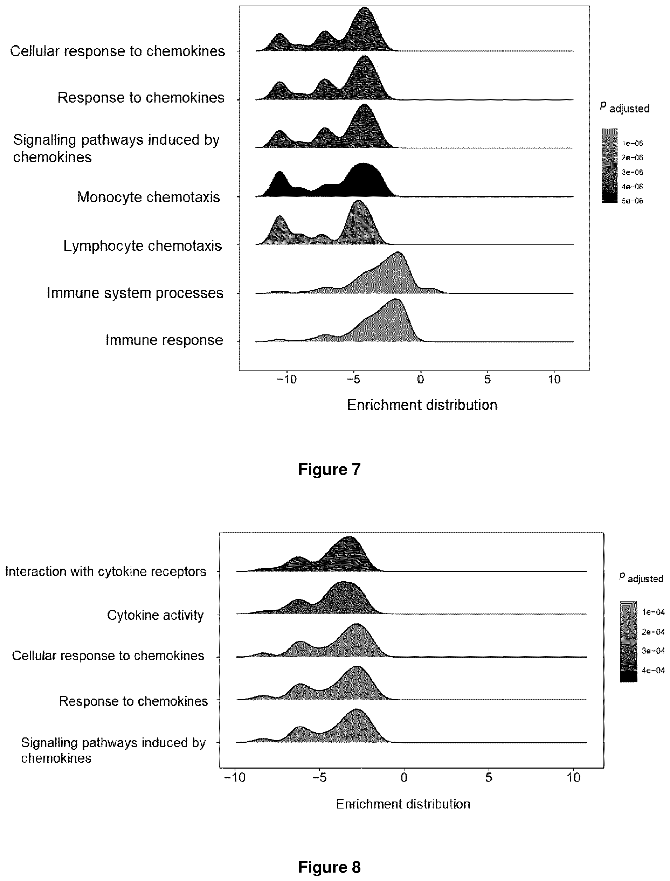

Figure 7 shows the effect of DCA-VPA treatment on the pathophysiological mechanisms of T-lymphocyte inflammation and immune response related to inflammatory and immune response genes in healthy patients. DCA-VPA, by inducing changes in the expression of genes related to inflammation or immune response, significantly inhibited cellular response to chemokines, general response to chemokines, chemokine-mediated signaling pathways, monocyte biological mechanisms of chemotaxis, lymphocyte chemotaxis, pathophysiological mechanisms of immune system processes and immune response. T-lymphocytes from 5 healthy male controls and from patients treated with a combination of 2 mM VPA and 5 mM DCA for 24 hours were analyzed. The effect of the treatment was assessed by comparing the expression of control genes with the gene expression of T-lymphocytes treated with DCA-VPA. The method of analysis was applied. The enrichment plot of gene sets/biological pathways was plotted using the R enrichplot v1.2 software package. The input data was obtained from the list of statistically significantly expressed genes obtained by differential gene expression analysis. Each biological pathway in the diagram (Figure 7) is represented on the y-axis, while the x-axis shows the enrichment score. Biological pathways with an enrichment score < 0 are inhibited and those with an enrichment score > 0 are activated.

### Effects of DCA-VPA treatment on pathophysiological mechanisms of inflammation and immune response of stimulated T-lymphocytes in healthy patients related to changes in gene expression

A group of stimulated T-lymphocytes from healthy men was compared: 1) control; and 2) stimulated and treated with a combination of DCA-VPA for 24 hours. T-lymphocytes were stimulated for 24 hours with calcium ionophore (calcium ionophore A23187 hemicalcium salt, (C9275); dose - 500 ng/ml; manufacturer Sigma-Aldrich) and PMA (phorbol 12-myristate 13-acetate (P8139); dose - 50 ng/ml; manufacturer Sigma-Aldrich ). The cells of the stimulated-treated T-lymphocytes group were treated with a combination of 2 mM VPA and 5 mM DCA. T-lymphocytes from control (n = 6) and treated healthy men (n = 6) were analyzed by gene sequencing to determine the expression of genes related to inflammation and immune response and to analyze the effect of DCA-VPA on the biological mechanisms and pathways of inflammation.

PMA directly activates protein kinase C, strongly induces extracellular and intracellular ROS, and induces the release of cytokines and chemokines. A23187 increases intracellular Ca²⁺ concentration by directly facilitating Ca²⁺ transport across the plasma membrane. Stimulation of human immune cells with A23187 and PMA can induce proinflammatory changes in cytokine and chemokine levels, and such cell stimulation is used in a model of inflammation, and drug inhibition of stimulated cells can protect them from inflammatory damage.

Sequencing data analysis showed that DCA-VPA treatment inhibited interaction with cytokine receptors, cytokine activity, cellular response to chemokines, response to chemokines, and chemokine-mediated signaling pathways (Figure 8).

### Impact of DCA-VPA on biological mechanisms of pathogenesis in adult patients with COVID-19

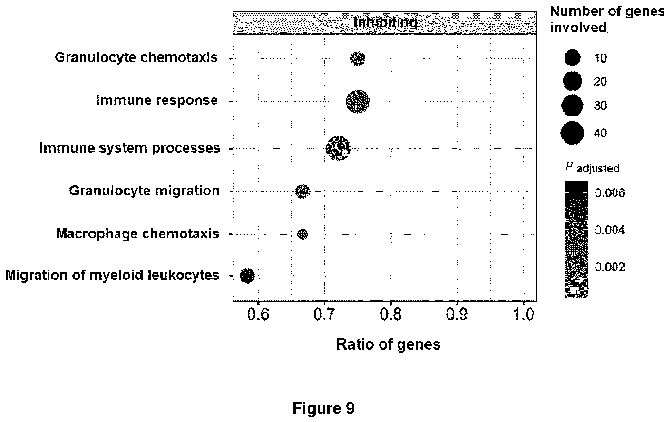

Gene set/biological pathway enrichment dot plot (Figure 9) shows the analysis of the effect of DCA-VPA on T-lymphocytes of men with severe COVID-19 (n = 3) using the R Enrichplot v1.2 software package. The input data were obtained from the list of statistically significantly expressed genes obtained by differential gene expression analysis. In this diagram, each biological pathway is represented on the y-axis and the x-axis represents the ratio of the number of differentially expressed genes in a gene set to the total number of genes in that gene set. Statistical significance values are marked in the legend according to the intensity of the monochrome color of the dots, respectively. T lymphocyte data from COVID-19 patients were analysed in the following groups: 1) T lymphocytes from control COVID-19 patients (n =3), 2) T lymphocytes from COVID-19 patients treated with DCA-VPA (n =3). Data from treated cells were compared to untreated cells. Cells were treated for 24 h with a mixture of 2 mM VPA and 5 mM DCA. Significant enrichment of gene sets related to inflammation and immune response was found in the following biological pathophysiological pathways: granulocytes chemotaxis, immune response, processes of the immune system, granulocyte migration, macrophages chemotaxis, migration of myeloid cells. The distribution of enrichment for gene sets related to inflammation and immune response ranged from -10 to -0.1, indicating that pro-inflammatory factors were significantly reduced, inhibited, either the treatment increased pro-inflammatory genes with increased expression of anti-inflammatory genes in the treated cell group. The study showed a significant inhibitory effect of DCA-VPA on inflammation and related immune response mechanisms.

The pathways shown in Figures 7, 8, and 9 were obtained by the gene set/biological pathway enrichment analysis method (Gene Set Enrichment Analysis; GSEA) using Enrichplot v1.2 and ClusterProfiler v4.8.2 R software packages.

## Claims

1. A pharmaceutical combination comprising an effective amount of (a) sodium dichloroacetate (DCA) and (b) valproic acid (VPA) or a pharmaceutically acceptable salt thereof, **characterized in that** said pharmaceutical combination is for use in the treatment of viral or bacterial infections or inflammatory diseases.

2. A pharmaceutical combination for use according to claim 1, **characterized in that** (a) the pharmaceutically acceptable salt of dichloroacetate is sodium dichloroacetate.

3. A pharmaceutical combination for use according to claim 1, **characterized in that** (b) the pharmaceutically acceptable salt of valproic acid is sodium valproate or magnesium valproate.

4. A pharmaceutical combination for use according to any one of claims 1 to 3, **characterized in that** said combination contains pharmaceutically acceptable excipients.

5. A pharmaceutical combination for use according to any one of claims 1 to 4, **characterized in that** the preparation is in any pharmaceutical form, such as a capsule, tablet or solution.

6. A pharmaceutical combination for use according to any one of claims 1 to 4, **characterized in that** dichloroacetate (DCA) and (b) valproic acid (VPA) or a pharmaceutically acceptable salt thereof are administered as a combined preparation or administered simultaneously as separate medicinal preparations.

7. A pharmaceutical combination for use according to any one of claims 1 to 6, **characterized in that** said viral or bacterial infections or inflammatory disease is selected from the group consisting of: typhoid and paratyphoid (A01), other salmonella infections (A02), shigellosis (A03), other bacterial intestinal infections (A04), pathogen infections caused by *Escherichia coli* (A04.0-A04.4), *Clostridium difficile* enterocolitis (A04.7), amoebiasis (A06), tuberculosis (A15-A19), extraintestinal yersiniosis (A28.2), pertussis (A37), meningococcal infection (A39), streptococcal sepsis (A40), other sepsis (A41), legionellosis (A48.1), staphylococcal infection (A49.0), Lyme disease (A69.2), atypical viral infections of the central nervous system (A81), mosquito-borne viral encephalitis (A83), tick-borne viral encephalitis (A84), herpes viruses (herpes simplex) infections (B00), herpes zoster (B02), measles (B05), acute hepatitis (B16), acute hepatitis C (B17.1), chronic viral hepatitis (B18), human immunodeficiency virus (HIV) disease (B20-B24), cytomegalovirus disease (B25), infectious mononucleosis (B27), malaria (B50-B54), leishmaniasis (B55), African trypanosomiasis (B56), Chagas disease (B57), toxoplasmosis (B58), influenza and pneumonia (J09-J18), COVID disease (U07.1), *Helicobacter pylori* infection (B96.81), human papillomavirus infection, SARS, MERS infections, infections caused by Gram-positive bacteria, infections caused by Gram-negative bacteria, inflammatory bowel diseases.

8. A pharmaceutical combination for use according to any one of claims 1 to 7, **characterized in that** said combination is administered as an adjunctive treatment to specific therapy for a known infection or inflammation caused by it.
